(19) Europäisches Patentamt
European Patent Office
Office européen des brevets

(11) **EP 2 245 982 A1**

(12) **EUROPEAN PATENT APPLICATION**

(43) Date of publication:
**03.11.2010 Bulletin 2010/44**

(51) Int Cl.:
***A61B 1/05*** *(2006.01)*

(21) Application number: **09275032.2**

(22) Date of filing: **30.04.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(71) Applicant: **BAE Systems PLC**
**6 Carlton Gardens**
**London SW1Y 5AD (GB)**

(72) Inventors:
• **Laycock, Leslie Charles**
**Chelmsford, Essex**
**CM2 8HN (GB)**

• **Culfaz, Ferhat**
**Chelmsford, Essex**
**CM2 8HN (GB)**

(74) Representative: **BAE SYSTEMS plc**
**Group IP Department**
**P.O. Box 87**
**Warwick House**
**Farnborough Aerospace Centre**
**Farnborough**
**Hampshire GU14 6YU (GB)**

(54) **Endoscopic method and device**

(57) An endoscope comprising an elongate endoscopic structure having a distal end, a source of modulated light, means for illuminating with the modulated light a field of view from the distal end, an array of sensors, means for delivering to respective said sensors light reflected from discrete parts of the field of view, means for determining the relative times of arrival of the reflected light at the respective sensors and means for deriving from the relative times of arrival the relative distances of the discrete parts of the field of view from the distal end of the endoscopic structure.

FIG 1

EP 2 245 982 A1

**Description**

This invention relates to endoscopic methods and devices particularly but not exclusively for use in the internal inspection of structures, plant and equipment and in other imaging applications where access to view a scene is limited to small apertures. An example of the former is the internal inspection of offshore platform structures.

A characteristic of such applications is that the field of view afforded by the endoscope may encompass objects at a considerable distance from the distal end of the endoscope, unlike in medical endoscopy where features in the field of view of the endoscope are always relatively close to it. This gives rise to difficulties in identifying objects in the field of view, and the present invention at least in its preferred embodiments is directed to this problem.

In one aspect, the invention provides an endoscopic method comprising illuminating a field of view from a distal end of a endoscope with modulated light, delivering reflected modulated light from discrete parts of the field of view to respective sensors in an array of such sensors, determining the relative times of arrival of the reflected light at the respective sensors and deriving from the relative times of arrival the relative distances of the discrete parts of the field of view from the distal end of the endoscope. The term "light" as used herein includes middle and near UV and near-infrared as well as visible light

The method may comprise determining the relative times of arrival by reference to phase differences between the reflected modulated light received at the respective sensors.

The light may be continuous wave light and maybe modulated sinusoidally.

In a second aspect, the invention provides an endoscope comprising an elongate endoscopic structure having a distal end, a source of modulated light, means for illuminating with the modulated light a field of view from the distal end, an array of sensors, means for delivering to respective said sensors light reflected from discrete parts of the field of view, means for determining the relative times of arrival of the reflected light at the respective sensors and means for deriving from the relative times of arrival the relative distances of the discrete parts of the field of view from the distal end of the endoscopic structure.

The elongate endoscopic structure may comprise a bundle of optical fibres, the array of sensors being disposed at the proximal end thereof, the optical fibres being configured to deliver the reflected light to the respective sensors.

The light source may be disposed at the proximal end of the endoscopic structure, the endoscope comprising further optical fibres to convey the modulated light of the distal end thereof.

The determining means may be configured to determine phase differences between the reflected modulated light received at the respective sensors.

The light source preferably provides continuous wave light and may be configured to provide sinusoidally modulated light.

The said sensors may be incorporated in a digital camera.

The endoscope may comprise a further camera and means for splitting light delivered by the optical fibres to the proximal end of the endoscope between the digital camera and the further camera.

The light-splitting means may be a dichroic mirror.

The endoscope may comprise means configured for presenting to an observer an image of the field of view which is formed using information regarding the said relative distances.

The image-presenting means maybe configured to combine the relative distance information with an image from the further camera.

The invention will be described merely by way of example with reference to the accompanying drawings wherein;

Figures 1 and 2 show an embodiment of the invention.

Figure 3 shows a camera used in the embodiment of Figures 1 and 2.

Figures 4 and 5 illustrate the operation of the camera of Figure 3, and

Figure 6 shows part of the camera of Figure 3.

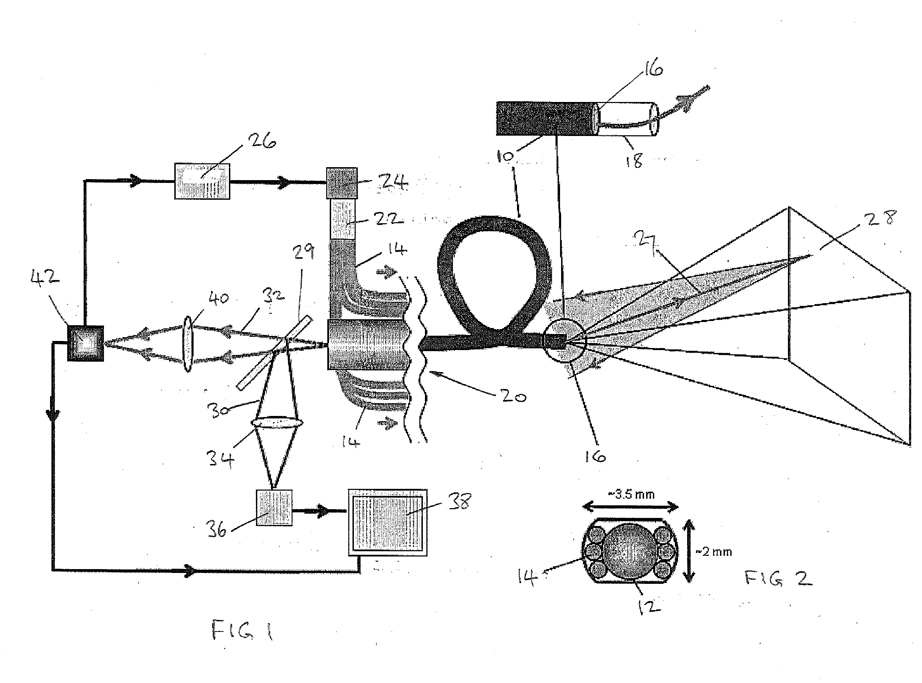

Referring to Figures 1 and 2, an endoscope apparatus comprises an elongate endoscopic structure 10 consisting of a two-part bundle of optical fibres. The main part is a bundle of fine imaging fibres 12 (Figure 2) of typical core diameter $4\mu m$. The bundle has about 30000 fibres and is of overall diameter 0.8mm. A suitable product is marketed by Sumitomo under the reference IGN-08/30. Bound up with the optical fibres so as to form a single flexible structure are a small number of relatively large illumination fibres 14, here shown for clarity as six, although a greater number could be employed if desired; in a prototype using a Swiss Ranger 4000 camera as described later, there are twenty six illumination fibres 14.

The distal end 16 of the imaging bundle 12 is terminated by a graded refractive index (GRIN) lens 18, e.g. from Grintech GmbH. The refractive index of the lens varies parabolically in a radial direction across the width of the lens, the maximum refractive index being at the centre of the lens and the minimum at the edge. This enables the fibres to focus and collect the light more efficiently resulting in a very compact system where the lens can be directly cemented to the fibre bundle. This generates an image of the scene being viewed onto the distal surface of the fibre bundle. A typical field of view is 60°. The distal ends of the imaging bundle fibres 12 and the illuminating fibres 14 are as far as possible co-planar. The lens 18 is glued to the fibres 12 by suitable glue of matching refractive index. In a prototype apparatus the refractive index of the centre of the GRIN lens is 1.636 and at the edge is ~1.33. The hyperfocal distance of the GRIN lens (diameter = 2mm, lens length = 5.29mm) is 30cm. Beyond this distance, all objects are in focus.

The proximal end of the endoscopic fibre bundle 10 is shown enlarged at 20 in Figure 1. The illumination fibres 14 are gathered together and their ends are glued to a collimating lens 22 to which is coupled an array of ultra-bright LEDs 24. In this example, some of the LEDs emit near-infrared light of wavelength 850nm, and the remainder emit white light.

The array of LEDS 24 are driven-in-phase with each other by a driver circuit 26 to produce continuous wave intensity-modulated light. Here the modulation is sinusoidal at 20MHz. All of the fibres 14 are of equal length, so the modulated light 27 emerging from the distal ends of each of the illumination fibres 14 is in phase with the light emerging from the other fibres 14. The modulated light illuminates a field of view 28 as seen by the totality of the imaging fibres 12. The light is reflected off objects in the field of view back to the imaging fibres 12. Each fibre individually has a very narrow field of view, and thus sees only a respective small part of the total field of view 28. Each fibre thus receives reflected light at a time dependent on the distance of the features in its field of view from the distal end 16 of the fibres 12, 14. The relative times of arrival at each fibre 12 thus can yield range information regarding the various features making up the total field of view. The difference in arrival times of the reflected light at the distal ends of the fibres 12 manifests itself as relative phase differences in the received light, as will be discussed further below.

The received light passes back along the imaging bundle 12 (the fibres of which are all of the same length) to the proximal end 20 thereof where it emerges and passes through a beam-splitting dichroic mirror 29 where the light is divided into two parts, a visible white-light component 30 and a near-infrared component 32. The visible component is taken via a lens 34 to a video camera 36 which has a pixel resolution at least equal to the number of fibres in the imaging bundle 12. The output of the video camera 36 is fed to a PC and display 38 to provide a high-definition but two dimensional image of the field of view.

The infrared component 32 is taken via a lens 40 to a range-finding video camera 42, where the light from each fibre 12 is processed as described below to extract its range data. As the 2D and 3D images correspond to the same field of view, the 2D image can be scaled and warped onto the 3D depth map in order to create a real-time 3D colour image of the field of view. If the 2D image is produced under infrared illumination, then the depth of object can be colour coded as a function of range.

The camera 42 conveniently may be a Swiss Ranger 4000 as sold by Mesa Imaging AG, Zurich, Switzerland (www.mesa-ioaging.ch) to whose website the reader is referred for further information. The following description is based on the paper "CCD/CMOS lock-in pixel for range imaging: challenges, limitations and state-of-the-art" by Büttgen et al, available on the website and incorporated herein by reference.

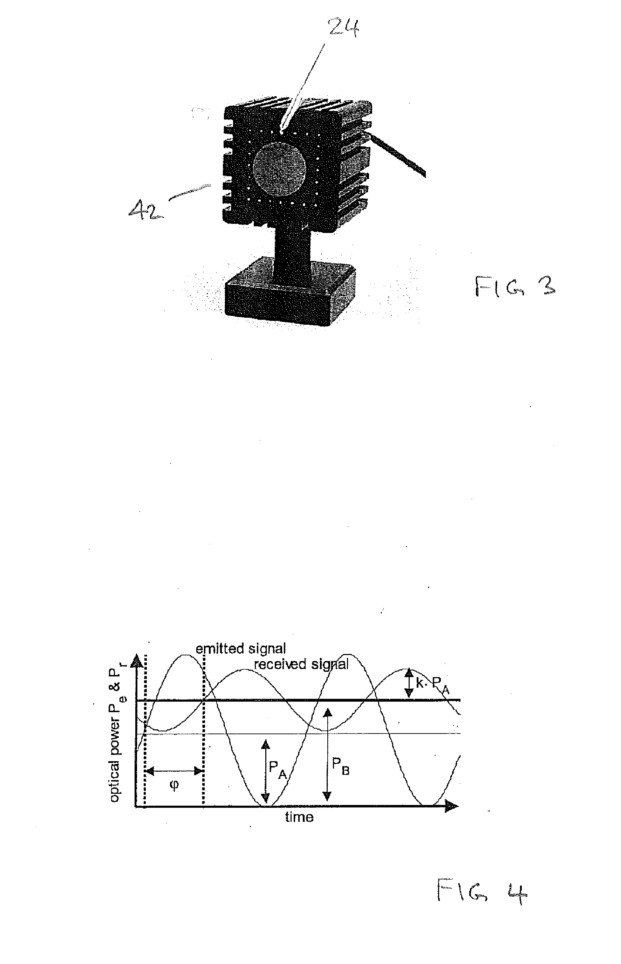

Referring to Figure 3, the camera 42 has an array of twenty-six LEDS 24 surrounding a lens 44, and incorporates within its circuitry the driver 26 (Figure 1) which is controlled by a common time base with the range-determination circuitry of the camera. The lens 40 (Figure 1) focuses an image of the proximal ends of the imaging fibre bundle 12 on an imaging CCD/CMOS array 44 of the camera 42. In the prototype there are more optical fibres 12 than there are pixels in the camera, so individual pixels will on average see more than one fibre 12. The camera 42 comprises three stacked printed circuit boards (PCB). The top PCB hosts a combined CCD/CMOS sensor, a power supply and the driver 26. The bias voltages and currents used to operate the sensors are implemented here as well as converting the signal into data which can be read by the next PCB module underneath, an image processing module. This module processes all the acquired data and calculates the 3D image range. The processed data are then transferred to a communication interface module PCB that interfaces to the PC 38.

Figure 4 shows the relationship between the illuminating light Pe emitted from the distal ends of the fibres 14 and the reflected light Pr received at the distal end of a fibre 12.

The phase shift $\varnothing$ is the result of the signal round-trip from the field of view and back. It is exploited for the extraction of the distance information.

The received signal is offset-shifted by a mean optical power $P_B$ mainly due to additional background light and a non-perfect demodulation. The offset component describes the intensity information.

The mean power of the emitted signal is $P_A$, and the amplitude of the reflected signal is reduced by a factor k, due to optical losses. The ratio of $kP_A$ to $P_B$ defines the signal-to-background ratio and hence the achievable accuracy is determined.

It can be shown by analogy from Büttgen, (op.cit) that the range R from the distal end 16 of the endoscopic structure 10 to a feature in the field of view of a fibre 12 is given by

$$R = c\emptyset / 4\pi f$$

Where c is the velocity of light and f is the modulation frequency.

In the SR4000 camera, $\emptyset$ is determined by naturally-sampling the incoming sinusoidally-modulated optical signal four times per modulation period with each sample shifted by 90 degrees. The modulation signal then can be reconstructed unambiguously. Each sample corresponds to the integration of the photo-generated charge carriers in the camera pixel over a fraction of the modulation period. The summation of each sample over several modulation periods increases the signal-to-noise ratio. For example using a modulation frequency of 20MHz and a summation over 10ms means that each sampling can be integrated over 200.000 modulation periods. Based on four samples *A0*, *A1*, *A2* and *A3,* the three signal parameters offset *B*, amplitude *A* and phase $\emptyset$ can be extracted:

$$\text{Offset} \qquad B = \frac{A0 + A1 + A2 + A3}{4}$$

$$\text{Amplitude} \qquad A = \sqrt{\frac{(A0 - A2)^2 + (A1 - A3)^2}{2}}$$

$$\text{Phase} \qquad \emptyset = \text{atan}\left(\frac{A0 - A2}{A1 - A3}\right)$$

*A* and *B* are represented by numbers of electrons. The offset describes the total intensity of the detected signal, also including background light, and the amplitude is a measure of the signal itself.

In each pixel of the camera 42, the integration of the photo-generated charges is performed over half of the modulation period for each of the four samples. Both the detection and the demodulation are done in the charge-domain using charge-coupled devices. That can provide almost noise-free demodulation of the light signal over a wide range of operating conditions.

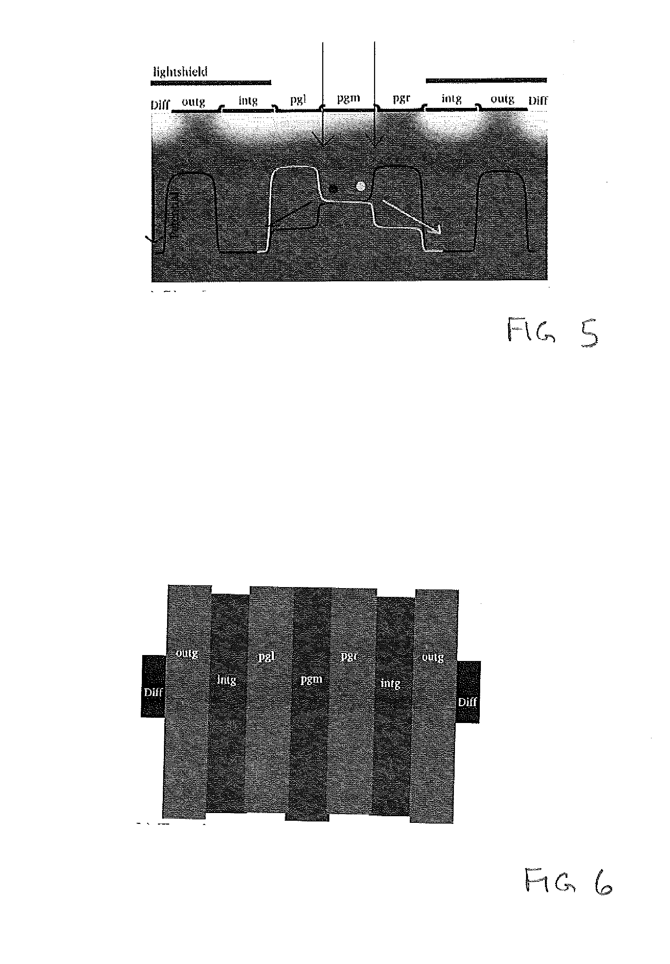

Figure 5 explains the pixel architecture and its sampling process. A symmetric sub-sequence of overlapping poly-silicon gates forms the demodulation pixel that delivers two samples of the impinging optical signal at the same time. The generation of the charges takes place below the left, right and middle photo-gates (PGL, PGR, PGM). Applying appropriate control voltages to the photo-gate, a characteristic potential distribution is built up in the buried channel forcing the charges to drift to the left or right side. On each side, the charges are stored below an integration gate (intg). The left and right photo-gates are toggled synchronously with the modulation frequency so that each charge packet below one integration gate corresponds to one sample of the light signal. In order to read out the charge packets, the voltage on the integration gates is decreased so that the accumulated charges will drop over the out gate (outg) into the sensing diffusion region (diff). Standard source follower circuits in complementary-metal-oxide-semiconductor (CMOS) technology, implemented within the pixel but not shown in Figure 5, provide the necessary amplification of the diffusion voltage for high speed read-out.

Figure 6 shows a top view of the pixel. In order to demodulate at frequencies of some tens of Megahertz, the transport paths of the photo-generated charges have to be kept as short as possible. As a consequence, the sensitive area can only be increased by stretching the gates in just one direction, as can be seen in the top view of the pixel.

The resolution of the Swiss Ranger camera for a target 5m away is 2.7cm in the x-direction and 2.4cm in the y-direction. The resolution in z is +/-1cm. The operating range of the Swiss Ranger in the Z-direction is 0.3m to 5m.

As described, the endoscope uses both near-infrared and visible light. It thus is suitable for industrial inspection but not for covert surveillance, because the visible light can be seen by the surveillance subject. For covert applications, the field of view can be illuminated using two sources of infrared light sufficiently different in wavelength to be discriminated by a suitable dichroic mirror 29. Alternatively the mirror 29, lens 34 and video camera 36 can be omitted and an image formed solely from near-infrared light supplied to the camera 42. Whilst the image quality may be somewhat reduced, it nevertheless should be adequate for surveillance applications, when for example the distal end 16 of the endoscope would be inserted through a small hole in a wall or even through a key-hole.

**Claims**

1. An endoscopic method comprising illuminating a field of view from a distal end of a endoscope with modulated light, delivering reflected modulated light from discrete parts of the field of view to respective sensors in an array of such sensors, determining the relative times of arrival of the reflected light at the respective sensors and deriving from the relative times of arrival the relative distances of the discrete parts of the field of view from the distal end of the endoscope.

2. The method of claim 1 comprising determining the relative times of arrival by reference to phase differences between the reflected modulated light received at the respective sensors.

3. The method of claim 1 or claim 2 comprising modulating the illuminating light sinusoidally.

4. An endoscope comprising an elongate endoscopic structure having a distal end, a source of modulated light means for illuminating with the modulated light a field of view from the distal end, an array of sensors, means for delivering to respective said sensors light reflected from discrete parts of the field of view, means for determining the relative times of arrival of the reflected light at the respective sensors and means for deriving from the relative times of arrival the relative distances of the discrete parts of the field of view from the distal end of the endoscopic structure.

5. The endoscope of claim 4 wherein the elongate endoscopic structure comprises a bundle of optical fibres, the array of sensors being disposed at the proximal end thereof, the optical fibres being configured to deliver the reflected light to the respective sensors.

6. The endoscope of claim 4 or claim 5 wherein the light source is disposed at the proximal end of the endoscopic structure, the endoscope comprising further optical fibres to convey the modulated light of the distal end thereof.

7. The endoscope of any of claims 4 to 6 wherein the determining means is configured to determine phase differences between the reflected modulated light received at the respective sensors.

8. The endoscope of any of claims 4 to 7 wherein the light source is configured to provide sinusoidally modulated light.

9. The endoscope of claims 7 and 8 comprising a digital camera incorporating the said sensors.

10. The endoscope of claim 5 and claim 9 comprising a further camera and means for splitting light delivered by the optical fibres to the proximal end of the endoscope between the digital camera and the further camera.

11. The endoscope of claim 10 wherein the light splitting means is a dichroic mirror.

12. The endoscope of any of claims 4 to 11 comprising means configured for presenting to an observer an image of the field of view which is formed using information regarding the said relative distances.

13. The endoscope of claim 12 when dependent from claim 10 or claim 11 wherein the image-presenting means is configured to combine the relative distance information with an image from the further camera.

FIG 1

FIG 2

FIG 3

FIG 4

FIG 5

FIG 6

Europäisches
Patentamt

European
Patent Office

Office européen
des brevets

## EUROPEAN SEARCH REPORT

**Application Number**

EP 09 27 5032

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/139920 A1 (SEIBEL ERIC J [US] ET AL SEIBEL ERIC J [US] ET AL) 3 October 2002 (2002-10-03) | 1,2,4,6, 7,9,12 | INV. A61B1/05 |
| Y | | 3,5,8, 10,13 | |
| | * paragraphs [0003], [0006], [0012], [0014] * <br> * paragraphs [0048] - [0056] * <br> * paragraphs [0124] - [0137] * <br> ----- | | |
| X | US 2008/013960 A1 (TEARNEY GUILLERMO J [US] ET AL) 17 January 2008 (2008-01-17) * paragraphs [0034] - [0038] * * paragraphs [0053] - [0056] * | 1,2,4-7, 9 | |
| Y | | 3,5,8, 10,13 | |
| | ----- | | |
| A | EP 1 714 607 A (GIVEN IMAGING LTD [IL]) 25 October 2006 (2006-10-25) * paragraph [0098] * ----- | 1-13 | |

**TECHNICAL FIELDS SEARCHED (IPC)**

A61B

The present search report has been drawn up for all claims

| Place of search | Date of completion of the search | Examiner |
|---|---|---|
| Munich | 5 October 2009 | Rivera Pons, Carlos |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 27 5032

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2009

| Patent document cited in search report | | Publication date | Patent family member(s) | | Publication date |
|---|---|---|---|---|---|
| US 2002139920 | A1 | 03-10-2002 | US | 6294775 B1 | 25-09-2001 |
| US 2008013960 | A1 | 17-01-2008 | AU | 3119802 A | 21-05-2002 |
| | | | EP | 1343411 A2 | 17-09-2003 |
| | | | WO | 0238040 A2 | 16-05-2002 |
| EP 1714607 | A | 25-10-2006 | JP | 2006297109 A | 02-11-2006 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Büttgen.** *CCD/CMOS lock-in pixel for range imaging: challenges, limitations and state-of-the-art*